Europäisches Patentamt

European Patent Office

Office européen des brevets

(11) Numéro de publication: **0 180 704**
**B1**

# (12) FASCICULE DE BREVET EUROPEEN

(45) Date de publication du fascicule du brevet: **12.10.88**

(51) Int. Cl.⁴: **C 07 C 126/02**

(21) Numéro de dépôt: **85104811.6**

(22) Date de dépôt: **20.04.85**

(54) Procédé perfectionné pour la production de l'urée.

(30) Priorité: **19.05.84 CH 2477/84**

(43) Date de publication de la demande:
**14.05.86 Bulletin 86/20**

(45) Mention de la délivrance du brevet:
**12.10.88 Bulletin 88/41**

(84) Etats contractants désignés:
**AT DE FR GB IT NL**

(56) Documents cités:
**EP-A-0 098 396**
**FR-A-2 389 601**
**GB-A-2 072 170**

**PATENT ABSTRACTS OF JAPAN, vol. 6, no. 118, 2 juillet 1982, page (C-111) (996); & JP-A-57-46954 (MITSUI TOATSU KAGAKU) 17-03-1982**

(73) Titulaire: **AMMONIA CASALE S.A.**
**Via della Posta 4**
**CH-6900 Lugano (CH)**

(73) Titulaire: **Zardi, Umberto**
**Via Lucino 57**
**CH-6932 Breganzona (Ti) (CH)**

(72) Inventeur: **Zardi, Umberto**
**Via Lucino 57**
**CH-6932 Breganzona (CH)**

(74) Mandataire: **Incollingo, Italo**
**AMMONIA CASALE S.A. Via della Posta 4**
**CH-6900 Lugano (CH)**

Courier Press, Leamington Spa, England.

# 0 180 704

**Description**

La présente invention concerne un procédé perfectionné pour la synthèse de l'urée à partir d'ammoniaque et d'anhydride carbonique à une pression et une température adéquates. La formation de l'urée se déroule dans une zone (ou des zones) de synthèse où un excès d'ammoniaque libre est maintenu afin de favoriser des taux de conversion élevés.

Ce procédé perfectionné comporte, en particulier, une nouvelle phase de traitement destinée à récupérer et à recycler les matières non transformées (ammoniaque libre et le carbamate) dans la zone (ou les zones) de réaction d'une façon optimale, en vue de réduire la consommation d'énergie et les coûts d'investissement.

Il est connu que des rendements de réaction élevés sont favorisés par un fort excès d'ammoniaque (par rapport à la valeur stoéchiométrique) mais exigent, cependant, une haute pression opérationnelle du réacteur et, par conséquent, des unités de traitement complexes et une importante consommation d'énergie en aval du réacteur, pour collecter et recycler l'excès d'ammoniaque et le carbamate résiduel contenus dans l'urée produite.

Récemment, certains procédés ont été mis au point pour réduire la consommation d'énergie et les coûts d'investissement requis pour les unités de traitement en aval du réacteur, mais ce sont là des procédés encore complexes qui impliquent une consommation d'énergie considérable.

Le brevet américain 4208347 (Montedison), connu comme le "IDR Process" (Isobaric Double Recycle), procédé à double recyclage isobare, décrit un système de traitement par stripping, à deux phases, selon lequel le carbamate est séparé à l'aide d'ammoniaque comme agent de dégazage (stripping agent) dans la première phase, tandis que l'ammoniaque libre est séparée en utilisant l'anhydride carbonique comme agent de dégazage ment dans la deuxième phase. Une certaine complexité de ce système est évidente.

Le brevet américain 4321410 (Mitsui Toatsu Chemicals and Toyo Engineering), connu comme le procédé ACES (Advanced Process for Cost and Energy saving), décrit un traitement par stripping à deux phases, exécuté dans un appareil de dégazage (stripper) nouvellement conçu, selon lequel l'effluent du réacteur d'urée entre en contact avec le gaz (principalement $NH_3$ et $CO_2$) sortant d'un échangeur de chaleur à pellicule tombante dans une première phase de traitement adiabatique où l'ammoniaque libre es séparée et successivement traitée dans un échangeur de chaleur à pellicule tombante (deuxième phase de traitement) à contre-courant de l'anhydride carbonique utilisé comme agent de dégazage pour éliminer le carbamate résiduel.

Selon ce procédé, la quantité d'ammoniaque libre qui peut être séparée de l'effluent sortant du réacteur est limitée en raison de la présence de $NH_3$ dans les gaz en contact avec la solution d'urée dans la phase adiabatique, tandis qu'une quantité minime d'ammoniaque libre dans la solution d'urée est souhaitable pour atteindre un résultat optimum dans la séparation du carbamate durant le phase successive de stripping au $CO_2$.

La demande de brevet italien 24357A/80 ce resp. un brev. anglars 2087381 (Snamprogetti) propose un procédé fort semblable au procédé Montedison mais avec deux phases de traitement à des pressions différentes (non isobares).

Aucun de ces nouveaux procédés ne permet le recyclage direct dans le réacteur de l'ammoniaque libre séparée de l'effluent sortant du réacteur, ce qui constitue un excellent moyen de minimiser les coûts d'investissement et la consommation d'énergie. Le recyclage indirect de l'ammoniaque dans les unités de traitement en aval du réacteur est réalisé à l'aide de solutions aqueuses, l'eau étant recyclée dans le réacteur, et cela crée un désavantage pour les rendements de la réaction.

Les procédés de la génération passée, suivis de ceux de la nouvelle génération, étaient dominés par les procédés de stripping au $CO_2$ de Stamicarbon, et au $NH_3$ de Snamprogetti qui comportent une seule phase de traitement à haute pression. Selon le procédé de stripping au $CO_2$ de Stamicarbon, l'effluent sortant du réacteur, avec une faible teneur en ammoniaque libre, est traité directement dans l'appareil de dégazage (stripper) au $CO_2$ pour en extraire le résidu de carbamate. La quantité d'ammoniaque dans le réacteur est maintenue à un niveau inférieur afin d'assurer une séparation optimum du carbamate dans l'appareil de dégazage au $CO_2$, mais les rendements de la réaction sont alors inférieurs et la consommation d'énergie et les coûts d'investissement sont considérables.

Selon le procédé de stripping au $NH_3$ de Snamprogetti, l'effluent sortant du réacteur, avec une teneur en ammoniaque plus importante, est également traitée dans une phase d'auto-dégazage (self-stripping) pour séparer le carbamate. Une quantité considérable d'ammoniaque libre est encore présente dans la solution d'urée sortant de l'appareil de dégazage et est recyclée séparément dans le réacteur au moyen de pompes. Ce système comporte l'emploi d'une colonne de rectification pour la séparation de l'ammoniaque pure et implique une consommation d'énergie et des coûts d'investissement relativement élevés. Aucun des procédés de la génération passée ne permet le recyclage direct dans le réacteur de l'ammoniaque libre récupérée à partir des effluents sortant du réacteur, moyennant une consommation d'énergie et des coûts d'investissement minimes.

Le recyclage direct d'importantes quantités d'ammoniaque dans la zone de réaction est un excellent moyen de réduire les coûts d'investissement et la consommation d'énergie, ce qui est l'objectif principal de la présente invention.

Il a été découvert, inopinément, que d'importantes quantités d'ammoniaque peuvent être séparées, d'une manière économique, à partir des effluents sortant de réacteurs fonctionnant avec un excès considérable d'ammoniaque et, par conséquent, des taux de conversion élevés, et un excès minimal d'ammoniaque dans la solution d'urée qui peut être traitée ultérieurement, d'une manière très efficace, dans un échangeur à pellicule tombante, avec un flux de $CO_2$ de dégazage en contre-courant, pour séparer le carbamate résiduel. Il est bien connu que la présence d'ammoniaque est nuisible à l'efficacité du dégazage au $CO_2$.

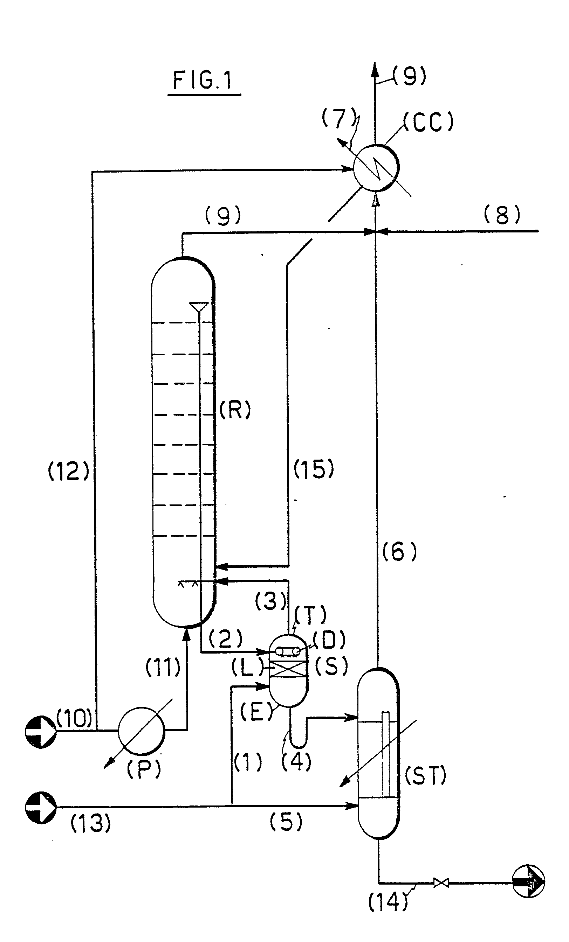

Le nouveau procédé est décrit en référence à la Figure 1 qui représente l'une des applications possibles de l'invention. La solution d'urée obtenue dans un réacteur (R) à taux de conversion élevé, donc avec l'apport d'un fort excès d'ammoniaque par rapport à la valeur stoéchiométrique, est traitée dans la phase adiabatique (S) où la majeure partie de l'excès d'ammoniaque est séparée grâce au contact intime qui intervient entre la solution d'urée et une quantité minime de $CO_2$ frais alimenté à la phase (S) par la conduite (1). La conduite (2) alimente la solution d'urée du réacteur (R), qui peut être de conception conventionnelle, à la phase (S), tandis que le recyclage direct de l'excès d'ammoniaque séparé de la phase (S) est assuré par la conduite (3). La solution d'urée, avec un excès minimal d'ammoniaque, sortant de la phase (S) est alors alimentée par le conduite (4) à un stripper au $CO_2$ (ST), également de conception conventionnelle, où le carbamate est séparé, d'une manière optimum, dans un échangeur à pellicule tombante avec un contre-courant de $CO_2$ frais comme agent de dégazage introduit dans la conduite (5).

Les vapeurs (principalement $NH_3$ et $CO_2$ provenant de la décomposition du carbamate) sortant du stripper (ST) sont alimentées par la conduite (6) au condenseur du carbamate (CC) dans lequel la chaleur produite est transformée en vapeur (conduite 7) qui est utilisée dans les phases de traitement conventionnelles (non représentées dans la Figure) de la solution d'urée en aval du stripper (ST).

Le condenseur du carbamate (CC) reçoit également le solution de carbamate (conduite 8) des phases detraitement susmentionnées et les substances inertes introduites avec le $CO_2$ qui sont déchargées du réacteur (R) (conduite 9). Après l'élimination des résidus de $NH_3$ et de $CO_2$ de condenseur de carbamate (CC), les substances inertes sont déchargées du système (conduite 9).

La charge d'ammoniaque (conduite 10) est en partie alimentée (conduite 11) au réacteur après avoir été préchauffée pour maintenir la balance de la chaleur du réacteur dans le système de préchauffage (P), et en partie alimentée au condenseur du carbamate (CC) (conduite 12). La conduite (13) distribue le $CO_2$ frais dans la majeure partie est alimentée au stripper (ST) (conduite 5) et une minorité à la phase (S) (conduite 1).

La solution d'urée (conduite 14), une fois traitée dans le stripper (ST), avec une quantité optimale de carbamate résiduel, est finalement alimentée aux phases de traitement conventionnelles (non représentées) pour obtenir le produit final (urée) désiré. La solution de carbamate est recyclée du condenseur de carbamate (CC) au réacteur par gravité (conduite 15).

Un contact intime d'une courte durée (quelques secondes) intervenant entre la solution d'urée avec un excès d'ammoniaque et le $CO_2$ frais alimenté à la phase adiabatique de séparation de l'ammoniaque, est critique.

Les Figures 1, 2 et 3, dans lesquelles une couche appropriée (L) (par exemple des anneaux ou des plateaux) pour le transfert de masse est prévue, représentent différentes applications de l'invention, notamment:

— Dans la Figure 1, la couche appropriée (L) pour le transfert de masse est introduite dans un appareil séparé (E) dont la partie supérieure fait fonction de séparateur pour la récupération de l'ammoniaque produite. Un distributeur de liquide (D) est aussi prévu.
— Dans la Figure 2, la phase de séparation de l'ammoniaque (S) est située dans la partie inférieure du réacteur (R) où la couche appropriée (L) pour le transfert de masse est introduite dans un espace vide (ES) dans la partie inférieure du réacteur.
— Dans la Figure 3, la phase de séparation de l'ammoniaque (S) est également située dans la partie inférieure du réacteur (R) où la couche appropriée (L) pour le transfert de masse est introduite dans un espace vide (ES) d'un diamètre réduit, dans la partie inférieure du réacteur.

La Figure 4 illustre l'application préférés de l'invention. Dans la phase adiabatique de séparation de l'ammoniaque (S) le contact intime entre la solution d'urée avec un excès d'ammoniaque (courant 2) et le $CO_2$ frait employé (courant 1) intervient en très peu de temps et provoque la transfert d'une masse considérable dans un mélangeur (VM) du type Venturi.

La vapeur d'ammoniaque ainsi dégagée est alors éliminée de la solution d'urée dans le séparateur (ES).

Les caractéristiques avantageuses de l'invention peuvent être illustrées par la comparaison faite ci-après entre la consommation d'énergie (consommation de vapeur dans le circuit de synthèse) des procédés de l'ancienne et la nouvelle générations, susmentionnés, et les consommations indiquées dans les exemples décrivant la présente invention.

Les chiffres concernant la consommation des procédés connus, ont été relevés de l'édition No. 143, mai 1983, de NITROGEN de Dooyeweerd and Messen:

| ACES Process: | 474 kg de vapeur à 22 bars pour 1000 kg d'urée |
| --- | --- |
| IDR Process (Montedison): | 524 kg de vapeur à 22 bars pour 1000 kg d'urée |
| $CO_2$ Stripping (Stamicarbon): | 633 kg de vapeur à 18 bars pour 1000 kg d'urée |
| Exemples 1 et 2: | 120 kg de vapeur à 22 bars pour 1000 kg d'urée |
| Exemple 3: | 150 kg de vapeur à 8 bars pour 1000 kg d'urée |

Les caractéristiques de l'invention sont mieux illustrées par les exemples suivants qui donnent la description du circuit de synthèse isobare. La même amélioration peut être obtenue dans des procédés où le stripper (ST) fonctionne à une pression inférieure à celle de la phase de séparation de l'ammoniaque (S).

Exemple 1

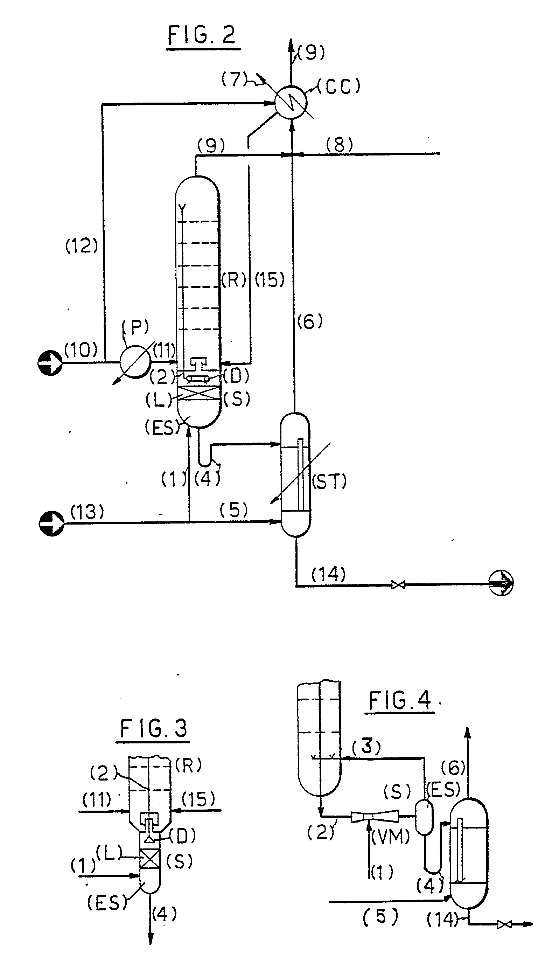

Figures 1, 2, 3, 4 (circuit de synthèse isobare)

Conditions opérationnelles de Réacteur (R)
— $NH_3/CO_2$ Rapport Molaire: 4.5
— $H_2O/CO_2$ Rapport Molaire: 0.4
— température: 188°C
— pression: 180 bar
— taux de conversion ($CO_2$ en urée): 74%

Composition et quantités des courants
— Courant (13) $CO_2$ frais: 45.833 kg (100°C)
— Courant (1) $CO_2$ frais à la
phase de séparation de l'ammoniaque (S): 4.375 kg (100°C)
— Courant (5) $CO_2$ frais au
stripper (ST): 41.458 kg (100°C)
— Courant (10) $NH_3$ frais: 35.417 kg (25°C)
— Courant (2) Solution d'urée
sortant du réacteur:

| $NH_3$ | 72.250 kg | 40.19% |
| --- | --- | --- |
| $CO_2$ | 16.125 kg | 8.97% |
| Urea | 62.500 kg | 34.77% |
| $H_2O$ | 28.875 kg | 16.07% |
| | 179.750 kg | 100.00% |

Température: 188°C

— Courant (4) Solution d'urée de la
phase de séparation de l'ammoniaque
(S) au stripper (ST):

| $NH_3$ | 29.750 kg | 21.17% |
| --- | --- | --- |
| $CO_2$ | 20.000 kg | 14.24% |
| Urea | 62.500 kg | 44.48% |
| $H_2O$ | 28.250 kg | 20.11% |
| | 140.500 kg | 100.00% |

Temperature: 191°C

— Courant (5) Recyclage direct de
l'ammoniaque dans le réacteur (R):

| $NH_3$ | 42.500 kg | 97.42% |
| --- | --- | --- |
| $CO_2$ | 500 kg | 1.14% |
| $H_2O$ | 625 kg | 1.44% |
| | 43.625 kg | 100.00% |

Temperature: 190°C

—Courant (14) Solution d'urée de stripper (ST):

| | | |
|---|---|---|
| NH$_3$ | 16.000 kg | 12.98% |
| CO$_2$ | 17.250 kg | 14.00% |
| Urea | 62.500 kg | 51.71% |
| H$_2$O | 27.500 kg | 22.31% |
| | 123.250 kg | 100.00% |

Temperature: 175°C

— Courant (6) Vapeurs de NH$_3$+CO$_2$ sortant du stripper (ST):

| | | |
|---|---|---|
| NH$_3$ | 13.750 kg | 23.43% |
| CO$_2$ | 44.208 kg | 75.30% |
| H$_2$O | 750 kg | 1.27% |
| | 58.708 kg | 100.00% |

Temperature: 190°C

— Courant (8) Solution de carbamate sortant des unités en aval:

| | | |
|---|---|---|
| NH$_3$ | 16.000 kg | 38.10% |
| CO$_2$ | 17.250 kg | 41.07% |
| H$_2$O | 8.750 kg | 20.83% |
| | 42.000 kg | 100.00% |

Consommation d'énergie
— Consommation de vapeur pour le stripper (ST): 120 kg de vapeur à 22 bars pour 1000 kg d'urée.

Dans les sections en aval du réacteur (non représentées dans la figure) du type conventionnel, le vapeur à 6—7 bar produite dans le condenseur de carbamate (CC) peut être utilisée pour la séparation et le recyclage des résidus de NH$_3$ et CO$_2$ présents dans la solution d'urée sortant du stripper au CO$_2$, avant la concentration finale, sous vide, de la solution d'urée en vue d'obtenir le produit fini.

E adoptant la technique "process to process" pour la récupération direct de la chaleur (système à effet multiple) aucune vapeur supplémentaire ne doit être importés des limites de batterie de l'installation.

Exemple 2
Figure 1, 2, 3, 4 (circuit de synthèse isobare)

Conditions opérationnelles du Réacteur (R)

| | |
|---|---|
| — NH$_3$/CO$_2$ Rapport Molaire: | 4.5 |
| — H$_2$O/CO$_2$ Rapport Molaire: | 0.4 |
| — température: | 188°C |
| — pression: | 180 bar |
| — taux de conversion (CO$_2$ en urée): | 74% |

Composition et quantités des courants

| | |
|---|---|
| — Courant (13) CO$_2$ frais: | 45.833 kg (100°C) |
| — Courant (1) CO$_2$ frais à la phase de séparation de l'ammoniaque (S): | 1.744 kg (100°C) |
| — Courant (5) CO$_2$ frais au stripper (ST): | 44.084 kg (100°C) |
| — Courant (10) NH$_3$ frais: | 35.417 kg (25°C) |

— Courant (2) Solution d'urée sortant de réacteur (R):

| | | |
|---|---|---|
| NH$_3$ | 72.250 kg | 40.19% |
| CO$_2$ | 16.125 kg | 8.97% |
| Urea | 62.500 kg | 34.77% |
| H$_2$O | 28.875 kg | 16.07% |
| | 179.750 kg | 100.00% |

Temperature: 188°C

5

| —Courant (4) Solution d'urée de la phase de séparation de l'ammoniaque (S) au stripper (ST): | $NH_3$<br>$CO_2$<br>Urea<br>$H_2O$ | 53.519 kg<br>17.669 kg<br>62.500 kg<br>28.687 kg | 32.96%<br>10.88%<br>38.49%<br>17.67% |
|---|---|---|---|
| | | 162.375 kg | 100.00% |

Temperature: 191°C

| —Courant (3) Recyclage direct de l'ammoniaque au réacteur (R): | $NH_3$<br>$CO_2$<br>$H_2O$ | 18.731 kg<br>200 kg<br>188 kg | 97.97%<br>1.05%<br>0.98% |
|---|---|---|---|
| | | 19.119 kg | 100.00% |

Temperature: 190°C

| —Courant (14) Solution d'urée sortant du stripper (ST): | $NH_3$<br>$CO_2$<br>Urea<br>$H_2O$ | 16.000 kg<br>17.250 kg<br>62.500 kg<br>27.500 kg | 12.98%<br>14.00%<br>50.71%<br>22.31% |
|---|---|---|---|
| | | 123.250 kg | 100.00% |

Temperature: 175°C

| —Courant (6) Vapeurs de $NH_3+CO_2$ sortant du stripper (ST): | $NH_3$<br>$CO_2$<br>$H_2O$ | 37.519 kg<br>44.508 kg<br>1.187 kg | 45.09%<br>53.49%<br>1.42% |
|---|---|---|---|
| | | 83.214 kg | 100.00% |

Temperature: 190°C

| —Courant (8) Solution de carbamate sortant des unités en aval: | $NH_3$<br>$CO_2$<br>$H_2O$ | 16.000 kg<br>17.250 kg<br>8.750 kg | 38.10%<br>41.07%<br>20.83% |
|---|---|---|---|
| | | 42.000 kg | 100.00% |

Consommation d'énergie
Voir Exemple 1.

Exemple 3

A propos des Figures 1, 2, 3 en 4 (circuit de synthèse isobare), par rapport à l'Exemple 2, les conditions opérationnelles ont été modifiées de sortie que le stripper (ST) puisse fonctionner dans des conditions adiabatiques. En l'occurence, le stripper (ST) pourrait être un appareil différent de l'échangeur à faisceau tubulaire (par ex. une colonne à plateaux), mais pour réduire le temps de séjour un appareil tubulaire à pellicule tombante pourrait être la meilleure solution, comme indiqué dans les figures.

Conditions opérationnelles de Réacteur (R)
| — $NH_3/CO_2$ Rapport Molaire: | 5 |
|---|---|
| — $H_2O/CO_2$ Rapport Molaire: | 0.5 |
| — température: | 190°C |
| — pression: | 200 bars |
| — taux de conversion ($CO_2$ en urée): | 76% |

6

Composition et quantités des courants
— Courant (13) $CO_2$ frais:

45.833 kg (100°C)

— Courant (1) $CO_2$ frais à la phase
de séparation de l'ammoniaque (S):

4.875 kg (100°C)

— Courant (5) $CO_2$ frais au
stripper (ST):

40.958 kg (100°C)

— Courant (10) $NH_3$ frais:

35.417 kg (25°C)

— Courant (2) Solution d'urée
sortant du réacteur:

| | | |
|---|---|---|
| $NH_3$ | 81.062 kg | 42.86% |
| $CO_2$ | 14.500 kg | 7.67% |
| Urée | 62.500 kg | 33.05% |
| $H_2O$ | 31.063 kg | 16.42% |
| | 189.125 kg | 100.00% |

Température: 190°C

— Courant (4) Solution d'urée de la
phase de séparation de l'ammoniaque
(S) au stripper (ST):

| | | |
|---|---|---|
| $NH_3$ | 29.750 kg | 21.03% |
| $CO_2$ | 18.875 kg | 13.35% |
| Urea | 62.500 kg | 44.19% |
| $H_2O$ | 30.313 kg | 21.43% |
| | 141.438 kg | 100.00% |

Temperature: 192°C

— Courant (9) Recyclage direct de
l'ammoniaque au réacteur (R):

| | | |
|---|---|---|
| $NH_3$ | 51.312 kg | 97.62% |
| $CO_2$ | 500 kg | 0.95% |
| $H_2O$ | 750 kg | 1.43% |
| | 52.562 kg | 100.00% |

Temperature: 191°C

— Courant (14) Solution d'urée du
réacteur (ST):

| | | |
|---|---|---|
| $NH_3$ | 20.625 kg | 15.24% |
| $CO_2$ | 22.500 kg | 16.63% |
| Urea | 62.500 kg | 46.19% |
| $H_2O$ | 29.688 kg | 21.94% |
| | 135.313 kg | 100.00% |

Temperature: 165°C

— Courant (6) Vapeurs de $NH_3+CO_2$
sortant du stripper (ST):

| | | |
|---|---|---|
| $NH_3$ | 9.125 kg | 19.38% |
| $CO_2$ | 37.333 kg | 79.29% |
| $H_2O$ | 625 kg | 1.33% |
| | 47.083 kg | 100.00% |

Temperature: 192°C

Consommation d'énergie
— Consommation de vapeur du stripper (ST)=Zéro
Dans les sections en aval du réacteur (non représentées dans la figure), du type conventionnel, la vapeur à 7—8 bars produite dans le condenseur de carbamate (CC) peut être utilisée pour la séparation et le recyclage des résidus de $NH_3$ et $CO_2$ contenus à un taux plus élevé dans la solution d'urée sortant du stripper au $CO_2$, avant la concentration finale, sous vide, de la solution d'urée en vue d'obtenir le produit fini.

En adoptant la technique "process to process" pour le récupération directe de la chaleur (système à effet multiple), une quantité réduite de vapeur à 8 bars, de l'ordre de 150 kg par 1000 kg d'urée, devra être importée des limites de batterie de l'installation.

Exemple 4
Cet exemple se réfère au procédé de stripping au $CO_2$ de Stamicarbon, de la génération passée, modifié selon la présente invention (Voir Fig. 1, 2, 3 et 4) dans le cas d'un projet de modernisation d'une

7

**0 180 704**

installation de stripping au $CO_2$ selon le procédé Stamicarbon, dans le but de réduire la consommation d'énergie.

Conditions opérationnelles du Réacteur (R)

| | |
|---|---|
| — $NH_3/CO_2$ Rapport Molaire: | 3.2 |
| — $H_2O/CO_2$ Rapport Molaire: | 0.4 |
| — Température: | 184°C |
| — Pression: | 145 bars |
| — Taux de conversion ($CO_2$ en urée): | 62% |

Composition et quantités des vapeurs

| | |
|---|---|
| — Courant (13) $CO_2$ frais: | 45.833 kg (100°C) |
| — Courant (1) $CO_2$ frais à la phase de séparation de l'ammoniaque (S): | 2.112 kg (100°C) |
| — Courant (5) $CO_2$ frais au stripper (ST): | 43.721 kg (100°C) |
| — Courant (10) $NH_3$ frais: | 35.417 kg (25°C) |

— Courant (2) Solution d'urée sortant du réacteur:

| | | |
|---|---|---|
| $NH_3$ | 56.000 kg | 31.55% |
| $CO_2$ | 28.125 kg | 15.84% |
| Urée | 62.500 kg | 35.21% |
| $H_2O$ | 30.875 kg | 17.40% |
| | 177.500 kg | 100.00% |

Température: 184°C

— Courant (4) Solution d'urée de la phase de séparation de l'ammoniaque (8) au stripper (S):

| | | |
|---|---|---|
| $NH_3$ | 44.250 kg | 26.44% |
| $CO_2$ | 30.037 kg | 17.95% |
| Urée | 62.500 kg | 37.34% |
| $H_2O$ | 30.575 kg | 18.27% |
| | 167.362 kg | 100.00% |

Température: 185°C

— Courant (3) Recyclage direct de l'ammoniaque au réacteur (R):

| | | |
|---|---|---|
| $NH_3$ | 11.750 kg | 95.92% |
| $CO_2$ | 200 kg | 1.63% |
| $H_2O$ | 300 kg | 2.45% |
| | 12.250 kg | 100.00% |

Température: 185°C

— Courant (14) Solution d'urée sortant du stripper (ST):

| | | |
|---|---|---|
| $NH_3$ | 9.133 kg | 8.22% |
| $CO_2$ | 10.846 kg | 9.77% |
| Urée | 62.500 kg | 56.28% |
| $H_2O$ | 28.575 kg | 25.73% |
| | 111.054 kg | 100.00% |

Température: 17=°C

— Courant (6) Vapeurs de $NH_3+CO_2$ sortant de stripper (ST):

| | | |
|---|---|---|
| $NH_3$ | 35.117 kg | 35.10% |
| $CO_2$ | 62.919 kg | 62.90% |
| $H_2O$ | 2.000 kg | 2.00% |
| | 100.036 kg | 100.00% |

Température: 185°C

— Courant (8) Solution de carbamate sortant des sections en aval:

| | | |
|---|---|---|
| $NH_3$ | 9.133 kg | 30.64% |
| $CO_2$ | 10.846 kg | 36.39% |
| $H_2O$ | 9.825 kg | 32.97% |
| | 29.804 kg | 100.00% |

Consommation d'énergie

La consommation de vapeur à 22 bars dans le circuit de systhèse (stripper au $CO_2$) est réduite dans la proportion de 100 kg par 1000 kg d'urée, en impliquant un investissement modeste pour l'installation de la section de séparation de l'ammoniaque (S).

Exemple 5

Cet exemple se réfère à l'application de la présente invention dans le revamping de procédés conventionnels sans stripping pour le recyclage total ou partiel, en vue de réduire la consommation d'énergie.

L'emploi de la phase de séparation et de recyclage direct de l'ammoniaque (S) pour traiter la solution d'urée sortant du réacteur, avant la première phase de décomposition, ne comportera qu'une faible quantité d'ammoniaque et, par conséquent, d'eau, à être recyclée dans les sections en aval avec, pour résultat, une amélioration des rendements de conversion du réacteur et une réduction de la vaporisation de l'éau.

Pour ces deux raisons (plus grands rendements de conversion et, par conséquent, moins de carbamate à recycler, et moins d'eau vaporisée) on peut obtenir une réduction de la vapeur entre 8 et 15 bars importée des limites de batterie, de l'ordre de 300 kg par 1000 kg d'urée.

Exemple 6

Cet exemple et réfère à l'application de l'invention dans le revamping des installations de stripping an $NH_3$ de Snamprogetti, pour réduire la consommation d'énergie et les coûts d'exploitation et d'entretien.

L'emploi de la phase de séparation et de recyclage de l'ammoniaque (S), dans le cas présent en aval du stripper, pour éliminer le fort excès d'ammoniaque du courant de solution d'urée traitée (un fort excès d'ammoniaque dans la solution d'urée sortant de réacteur facilite la séparation du carbamate par l'auto-dégazage au $NH_3$ dans le stripper) ne comportera qu'une faible quantité d'ammoniaque à être recyclée dans les sections en aval. On évite ainsi l'emploi d'une colonne de rectification pour la séparation et le recyclage de l'ammoniaque pure, à des coûts élevés et une importante consommation d'énergie.

**Revendications**

1. un procédé de production d'urée par synthèse à partir de l'ammoniaque et l'anhydride carbonique, à une pression et une température adéquates, où la formation de l'urée se produit dans une zone (ou des zones) de synthèse dans laquelle un excès d'ammoniaque est maintenu en vue de favoriser des taux de conversions élevés, laquelle zone (ou zones) de synthèse est suivie d'une phase de stripping au $CO_2$ durant laquelle le carbamate résiduel est séparé par un flux de $CO_2$ frais à contre-courant; le perfectionnement de ce procédé est caractérisé par l'emploi d'une phase de séparation de l'ammoniaque et son recyclage direct dans le réacteur, phase située entre la zone (ou les zones) de réaction et la phase de stripping au $CO_2$, la solution d'urée sortant de la zone (ou les zones) de réaction dans la phase de séparation étant en contact intime, pendant une courte durée, avec une minorité du $CO_2$ frais.

2. Un procédé selon la revendication 1, où le contact intime d'une courte durée entre la solution d'urée sortant d'une zone (ou des zones) de réaction et une minorité du $CO_2$ est obtenu dans un mélangeur du type Venturi dans lequel le flux de $CO_2$ frais s'écoule à co-courant de la solution d'urée.

3. Un procédé selon la revendication 1, où le contact intime d'une courte durée entre la solution d'urée sortant d'une zone (ou des zones) de réaction et une minorité du $CO_2$ est obtenu par l'apport d'une couche appropriée (des anneaux ou des plateaux) pour le transfert de masse, à travers laquelle passe le $CO_2$ à contre-courant de la solution d'urée.

4. Un procédé selon les revendications 1 et 2, où la durée du contact intime entre la solution d'urée et le $CO_2$ frais est inférieure à 10 secondes.

5. Un procédé selon les revendications 1 et 3, où la durée du contact intime entre la solution d'urée et le $CO_2$ frais est inférieure à 10 secondes.

6. Un procédé selon les revendications 1 à 5, où la portion de $CO_2$ frais alimentée à la phase de séparation de l'ammoniaque est égale ou inférieure à 20% de la totalité de $CO_2$, de preférence entre 4 et 12% de ladite totalité de $CO_2$ frais.

7. Un procédé, selon les revendications 1 à 6, où la phase de stripping au $CO_2$ est réalisée dans des conditions adiabatiques et la chaleur nécessaire pour éliminer le carbamate est fournie par la chaleur de réaction d'une partie du $CO_2$ frais en réaction avec l'ammoniaque libre pour former le carbamate.

8. Un procédé, selon les revendications 1 à 7, où la zone (ou les zones) de synthèse fonctionnent à des pressions variant entre 120 et 250 kg/cm².

9. Un procédé selon les revendications 1 à 8, où le rapport molaire $NH_3/CO_2$ dans la zone (ou les zones) de synthèse varie entre 2.5 et 6.

10. Un procédé selon les revendications 1 à 9 où la phase de stripping au $CO_2$ s'opère à une pression inférieure à celle de la phase de séparation de l'ammoniaque.

11. Un procédé de production d'urée par synthèse à partir de l'ammoniaque et l'anhydride carbonique, à une pression et une température adéquates, où la formation de l'urée se produit dans une zone (ou des zones) de synthèse dans laquelle un excès d'ammoniaque est maintenu en vue de favoriser des taux de

**0 180 704**

conversions élevés, laquelle zone (ou zones) étant suivie par une phase d'auto-dégazage au NH₃ durant laquelle le carbamate résiduel est séparé par un échangeur à pellicule tombante; le perfectionnement de ce procédé est caractérisé par l'emploi d'une phase de séparation et de recyclage direct de l'ammoniaque située en aval de la phase d'autodégazage au NH₃, la solution d'urée sortant de la phase d'autodégazage dans ladite phase de séparation étant en contact intime, pendant une courte durée, avec une minorité de CO₂.

12. Un procédé de production d'urée par synthèse à partir de l'ammoniaque et l'anhydride carbonique, à une pression et une température adéquates, où la formation de l'urée se produit dans une zone (ou des zones) de synthèse dans laquelle un excès d'ammoniaque libre est maintenu en vue de favoriser des taux de conversion élevés, laquelle zone (ou zones) de synthèse étant suivie d'un système de recyclage à phase multiple pour la matière résiduelle non transformée; le prefectionnement de ce procédé est caractérisé par l'emploi d'une phase de séparation et de recyclage directe de l'ammoniaque située en aval de la zone (ou des zones) de synthèse, la solution d'urée sortant de la zone (ou des zones) de synthèse dans ladite phase de séparation étant en contact intime, pendant une courte durée, avec une minorité du CO₂ frais.

**Patentansprüche**

1. Ein Herstellungsverfahren von Harnstoff durch Synthese ausgehend von Ammoniak und Kohlendioxid, bei angemessenen Druck- und Temperaturverhältnissen, wobei die Harnstoffbildung stattfindet in einer (oder mehreren) Synthesezone, in welcher ein Harnstoffüberschuss gehalten wird, um hohe Ronversionssätze zu begünstigen, welche Zone (oder Zonen) der Synthese gefolgt ist von einer $CO_2$-Strippingphase, während der das Restcarbamat getrennt wird durch einen Fluss von frischem Kohlendioxid im Gegenstrom; die Perfektionierung dieses Verfahrens ist gekennzeichnet durch den Einsaz einer Ammoniak-Trennungsphase und seiner direkte Wiedergewinnung im Reaktor, eine Phase die sich befindet zwischen der (oder den) Reaktionszone und der $CO_2$-Strippingzone, die Harnstofflösung tritt aus der (den) Reaktionszone in die Trennungsphase, durch engen Kontakt, während kurzer Dauer, mit einer Minorität des Frisch-$CO_2$.

2. Ein Verfahren, nach Anspruch 1, wo der enge Kontakt kurzer Dauer zwischen einer Harnstofflösung, die aus einer (oder mehreren) Reaktionszone tritt, und einer Minorität an $CO_2$, erreicht wird durch einen Venturi-Mischer, in welchem der Fluss von Frisch-$CO_2$ im Gleichstrom von der Harnstofflösung abläuft.

3. Ein Verfahren, nach Anspruch 1, wo der enge Kontakt kurzer Dauer zwischen der Harnstofflösung, die aus einer (oder mehreren) Reaktionszone tritt und einer Minorität en $CO_2$, erreicht wird durch die Beibringung einer geeigneten Lage (der Ringe und Platten) für den Massentransfer, durch die das $CO_2$ durchfliesst im Gegenstrom der Harnstofflösung.

4. Ein Verfahren, nach den Ansprüchen 1 und 2, wo die Dauer des engen Kontakts zwischen der Harnstofflösung und dem Frisch-$CO_2$ kleiner ist als 10 Sekunden.

5. Ein Verfahren, nach den Ansprüchen 1 und 3, wo die Dauer des engen Kontakts zwischen der Harnstofflösung und dem Frisch-$CO_2$ kleiner ist als 10 Sekunden.

6. Ein Verfahren, nach den Ansprüchen 1 bis 5, wo das Verhältnis von Frisch-$CO_2$, das der Ammoniak-Trennungsphase zugeführt wird, ≈20% des ganzen $CO_2$ ist; vorzugsweise zwischen 4 und 12% des erwähnten Totals an Frisch-$CO_2$.

7. Ein Verfahren, nach den Ansprüchen 1 bis 6, wo die $CO_2$, Strippingphase realisiert wird bei adiabatischen Bedingungen und wo die nötige Wärme, um das Carbamat zu eliminieren, geliefert wird durch die Reaktionswärme eines Teils des Frisch-$CO_2$ in Reaktion mit dem freien Ammoniak um das Carbamat zu bilden.

8. Ein Verfahren, nach den Ansprüchen 1 bis 7, wo die (oder die) Synthesezone funktioniert bei Drücken, die zwischen 120 und 250 kg/cm² variieren.

9. Ein Verfahren, nach den Ansprüchen 1 bis 8, wo das Molarverhältnis $NH_3/CO_2$ in der (oder den) Synthesezone variiert zwischen 2,5 und 6.

10. Ein Verfahren, nach den Ansprüchen 1 bis 9, wo man die $CO_2$-Strippingphase ausführt bei kleinerem Druck als dem der Ammoniak-Trennungsphase.

11. Ein Herstellungsverfahren von Harnstoff durch Synthese ausgehend von Ammoniak und Kohlendioxid, bei angemessenen Druck- und Temperaturverhältnissen, wo die Harnstoffbildung stattfindet in einer (oder mehreren) Synthesezone, in der ein Harnstoffüberschuss gehalten wird, um hohe Konversionssätze zu begünstigen, welche zone (oder zonen) der Synthese gefolgt wird von einer Zone der Auto-Entgasung mit Ammoniak, während der das Restcarbamat getrennt wird durch einen Tauscher mit fallendem Häutchen; die Perfektionierung dieses Verfahrens ist gekennzeichnet durch den Einsaz einer direkten Trennungs- und Wiedergewinnungsphase des Ammoniaks, gelegen im Abwärtsfluss der Auto-Entgasungsphase mit Ammoniak, die Harnstofflösung tritt aus der Auto-Entgasungsphase in besagter Trennungsphase, durch engen Kontakt, während kurzer Dauer, mit einer Minorität an $CO_2$.

12. Ein Herstellungsverfahren von Harnstoff durch Synthese ausgehend von Ammoniak und Kohlendioxid, bei angemessenen Druck- und Temperaturverhältnissen, wobei die Harnstoffbildung stattfindet in einer (oder mehreren) Synthesezone, in der ein Ueberschuss an freiem Ammoniak gehalten wird, um hohe Konversionssätze zu begünstigen, welche Zone (oder Zonen) der Synthese gefolgt ist von einem Wiedergewinnungssystem, das mehrphasig ist, für die nicht transformierte Restmaterie; die Perfektionierung dieses Verfahrens ist gekennzeichnet durch den Einsatz einer direkten Phase der Ammoniaktrennung- und Wiedergewinnung, gelegen im Abwärtsfluss der (oder der) Synthesezone, die Harnstofflösung tritt aus der (oder den) Synthesezone in besagter Trennungsphase durch engen Kontakt, während kurzer Dauer, mit einer Minorität des Frisch-$CO_2$.

**Claims**

1. System for the production of urea through synthesis from ammonia and carbon-dioxide, at an adequate pressure and temperature, where the formation of urea is produced in a synthesis zone (or zones) in which an excess of ammonia is maintained in order to facilitate high conversion rates, such synthesis zone (or zones) being followed by a $CO_2$ stripping phase during which the residual carbamate is separated by a fresh $CO_2$ flow in countercurrent; the improvement of this system is characterized by the use of an ammonia separation step and its direct recycle to the converter, above separation step being placed between the reaction zone (or zones) and the $CO_2$ stripping step, where the urea solution leaving the reaction zone (or zones) is in the above separation step put in direct contact with a minor portion of $CO_2$ feed for a short duration.

2. System according to claim 1, where close contact over a short period between the urea solution exiting a reaction zone (or zones) and a $CO_2$ minor portion is obtained in a mixing device Venturi type in which the fresh $CO_2$ flow flows into the urea solution co-currently.

3. System according to claim 1, where the close contact over a short period between the urea solution exiting a reaction zone (or zones) and a $CO_2$ minor portion is obtained by the contribution of an adequate layer (of rings or plates) for mass transfer, through which the $CO_2$ passes countercurrently to the urea solution.

4. System according to claims 1 and 2, where the duration of the close contact between the urea solution and the fresh $CO_2$ is less than 10 seconds.

5. System according to claims 1 and 3, where the duration of the close contact between the urea solution and the fresh $CO_2$ is less than 10 seconds.

6. System according to claims 1 to 5, where the portion of fresh $CO_2$ fed during the ammonia separation phase is equal or less than 20% of all of the $CO_2$, preferably between 4 and 12% of all of the above-mentioned fresh $CO_2$.

7. System according to claims 1 to 6, where the $CO_2$ stripping phase is carried out under adiabatic conditions and the heat necessary to eliminate the carbamate is supplied by the reaction heat of a fresh $CO_2$ portion in reaction with the free ammonia to form the carbamate.

8. System according to claims 1 to 7, where the synthesis zone (or zones) operates at pressures varying between 120 and 250 kg/cm$^2$.

9. System according to claims 1 to 8, where the $NH_3/CO_2$ molar ratio in the synthesis zone (or zones) varies between 2.5 and 6.

10. System according to claims 1 to 9, where the $CO_2$ stripping phase operates at a lower pressure than that of the ammonia separation phase.

11. System for the production of urea through synthesis derived from ammonia and carbon-dioxide, at an adequate pressure and temperature, where the formation of urea is produced in a synthesis zone (or zones) in which an excess of ammonia is maintained in order to facilitate high conversion rates, such zone (or zones) being followed by a $MH_3$ auto-degassing phase during which the residual carbamate is separated by a falling film exchanger; the improvement of this system is characterized by the use of a separation phase and the direct recyclement of ammonia located downstream to the $NH_3$ auto-degassing phase, the urea solution in the above-mentioned separation phase being in close contact, for a short period, with a $CO_2$ minor portion.

12. System for the production of urea through synthesis deriving from ammonia and carbon-dioxide, at an adequate pressure and temperature, where the formation of urea is produced in a one synthesis zone (or zones) in which an excess of ammonia is maintained in order to facilitate high conversion rates, such synthesis zone (or zones) being followed by a multiple phase recycle system for the untransformed residual material; the improvement of this system is characterized by the use of a separation phase and of direct recycle of the ammonia located downstream to the synthesis zone (or zones), the urea solution exiting the synthesis zone (or zones) in the above-mentioned separation phase being in close contact, during a short period, with a fresh $CO_2$ minor portion.

FIG.1

**0 180 704**

## FIG. 2

## FIG. 3

## FIG. 4

2